(19) Europäisches Patentamt
European Patent Office
Office européen des brevets

(11) Publication number: **0 171 133 B1**

(12) **EUROPEAN PATENT SPECIFICATION**

(45) Date of publication of patent specification: 28.08.91  (51) Int. Cl.⁵: **G01N 27/22**, G01R 27/26, G01N 27/04, G01N 33/10

(21) Application number: 85302290.3

(22) Date of filing: 02.04.85

(54) Electric moisture meter.

(30) Priority: 31.07.84 JP 160991/84

(43) Date of publication of application:
12.02.86 Bulletin 86/07

(45) Publication of the grant of the patent:
28.08.91 Bulletin 91/35

(84) Designated Contracting States:
DE FR GB

(56) References cited:
US-A- 3 761 810     US-A- 3 794 911
US-A- 3 961 247     US-A- 4 259 632
US-A- 4 278 935     US-A- 4 408 128

SOVIET INVENTIONS ILLUSTRATED, section E1, week KO3, March 2nd, 1983 DERWENT PUBLICATIONS LTD., London SO3

SOVIET INVENTIONS ILLUSTRATED, section E1, week E27, August 18th, 1982 DERWENT PUBLICATIONS LTD., London SO3

SOVIET INVENTIONS ILLUSTRATED, section R, week A51, February 7th, 1979 DERWENT PUBLICATIONS LTD., London R16

(73) Proprietor: KETT ELECTRIC LABORATORY
8-1, Minami-Magome-1-chome
Ota-ku Tokyo(JP)

(72) Inventor: Kuni, Toki
1434, Sannocho-1-chome Kamimaruko
Nakahara-ku Kawasaki-shi(JP)
Inventor: Hideyasu, Yuuki
568-11-2-505, Tsukui
Yokosuka-shi(JP)

(74) Representative: Miller, Joseph et al
J. MILLER & CO. Lincoln House 296-302 High Holborn
London WC1V 7JH(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to electric moisture meters for measuring the water content of grain, and particularly to an electric moisture meter capable of correcting measured water content for the error due to the change of weight of each grain sample from a standard weight.

An electric moisture meter is disclosed in the U.S. Patent No. 4,408,128 which was issued on Oct. 4, 1983 to Fujita and assigned to the same assignee as in this invention. An electric moisture meter measures an electric capacitance of an object being measured by holding it between the plus and minus electrodes, and indicates a value of the water content on the basis of the previously examined correlation between the water content and the electric characteristic.

A sample of an object to be measured, or for example, grain is placed in a cup of a constant volume and then poured into a moisture-meter grain container with two electrode plates provided as side walls. The change of electric capacitance is measured between the electrodes and converted to the water content of the grain.

This capacitance change depends on the weight and water content of the grain sample. However, the weight of grain filled in a constant-volume cup is not definite for every samples, or depends on the shape and size of a grain each of which varies for different kind of grain, and on the degree of packing the grain, or volume density.

If a constant weight of grain is sampled and placed in the moisture meter container for the measurement of water content, the moisture meter needs a balance and thus it takes much time to measure as compared with the measurement for a constant volume of grain placed in a cup.

If a measurement system is employed in which the sample container suspended by a spring actuates a micro-switch when it reaches a constant weight as disclosed in the U.S. Patent No. 3,794,911, it is necessary to delay the actuation of the switch so that the switch is not actuated by the inertia of the grain of short weight due to the pouring force of the sample grain. The short weight will result in a low value of water content being indicated.

US-A-3794911 discloses the features a) and b) of claim 1.

Accordingly, it is an object of the invention to provide an electric moisture meter which measures the water content of the sample on the basis of the electric capacitance of the sample placed between a pair of electrodes and is capable of correcting measured rate of water content for the error due to the variation of weight of each sample from a standard weight.

According to this invention, there is provided an electric moisture meter capable of correcting the measured water content value of each sample which is indicated on the base of the electric capacitance of the sample placed in an electric field, or between a pair of electrodes, for the error due to the change of weight of each sample from a standard weight by an electric signal produced in proportion to the weight of the sample, thereby reducing the scattering of density of each sample.

The present invention will be apparent from the following detailed description taken in conjunction with the accompanying drawings, in which:

Fig. 1 is a graph of the relation between the sample weight and the electric capacitance;

Fig. 2 is a graph of the output from a conventional moisture meter;

Fig. 3 is a circuit block diagram of a moisture meter of the invention;

Fig. 4 is a graph of the output from the moisture meter of the invention;

Fig. 5 is a schematic diagram showing the construction of a load-capacitance converter 13;

Fig. 6 is a circuit block diagram of one embodiment of a moisture meter according to this invention; and

Figs. 7a and 7b are detailed circuit diagrams of examples of an oscillator 14.

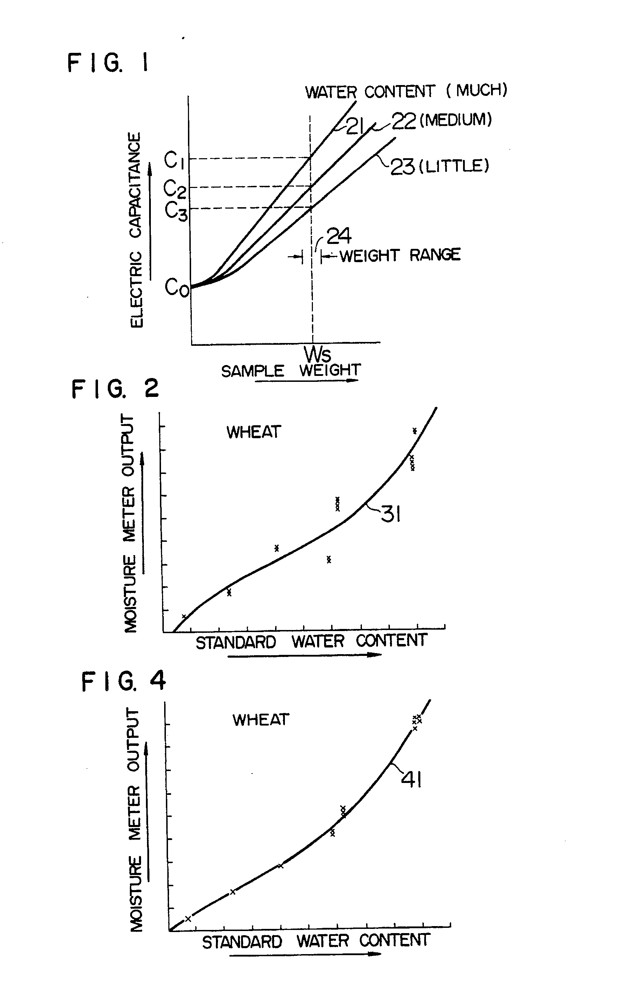

Fig. 1 shows the relation between the weight of a sample of grain and the electrical capacitance between the electrodes of an electric moisture meter. From Fig. 1, it will be seen that grain of more water content as indicated by a curve 21 has larger capacitance than the others indicated by curves 22 and 23. The capacitance $C_0$ at zero weight as shown in Fig. 1 depends on the electrode structure of the moisture meter. When grain of a constant volume is repetitively sampled, the weight of the samples scatters over a weight range 24 around a standard weight $W_s$ as shown in Fig. 1. The curves 21 to 23 are all substantially linear in the range 24 as illustrated. Therefore, the standard weight $W_s$ is necessary to be established at a high value because the curves 21 to 23 are not linear in a range of low values. In the moisture meter of this invention, the standard weight $W_s$ is selected to be 200 grams.

In Fig. 2, the abscissa shows the standard moisture content specified by the standard measurement (in the regulation) under the Japanese examination law for agricultural products, or the rate of weight reduced after grain (wheat) is left for 5 hours in the evironment of 105°C and one atmosphere, and the ordinate shows the output, or the rate of water content from a conventional moisture meter. A curve 31 is an imaginary curve determined by the minimum square method on the basis of the measured data which are much deviated from the curve 31 as indicated by small crosses.

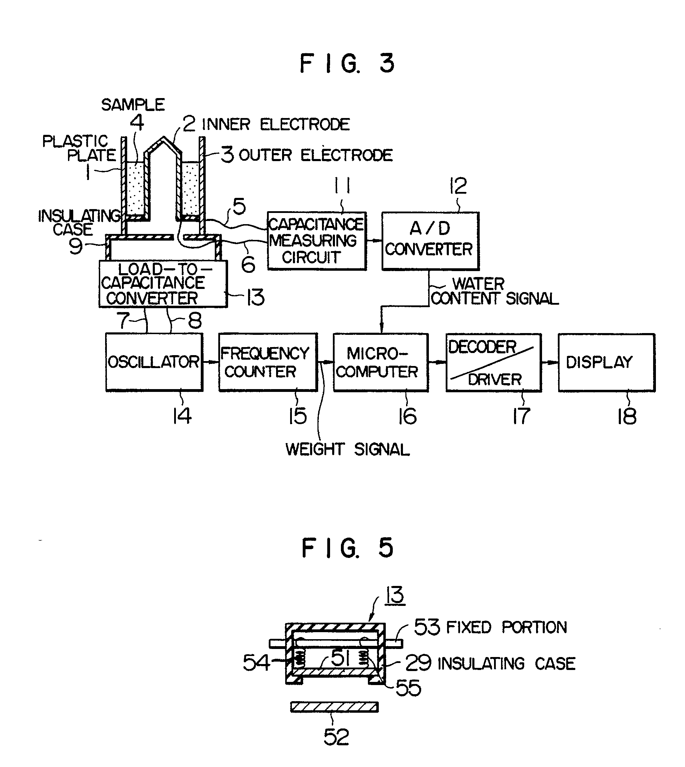

Fig. 3 shows an embodiment of a high-frequency electric moisture meter of the invention. A grain sample 4 in a constant-volume cup is poured into a sample container of the moisture meter, or placed in the space surrounded by an insulating bottom plate 1 made of for example, plastic, an inner electrode 2 and an outer electrode 3. The electrodes 2 and 3 are connected through wires 5 and 6 to a capacitance measuring circuit 11.

The electric capacitance is converted to a voltage corresponding to the moisture content by the capacitance measuring circuit 11, and then converted to a digital signal by an analog-to-digital converter 12. The digital signal is supplied to a microcomputer 16. The total weight of the sample container, or of the electrodes 2, 3, plate 1 and insulating case 9 is converted by a load-to-capacitance converter 13 to an electric capacitance, which is then supplied through wires 7, 8 to an oscillator 14.

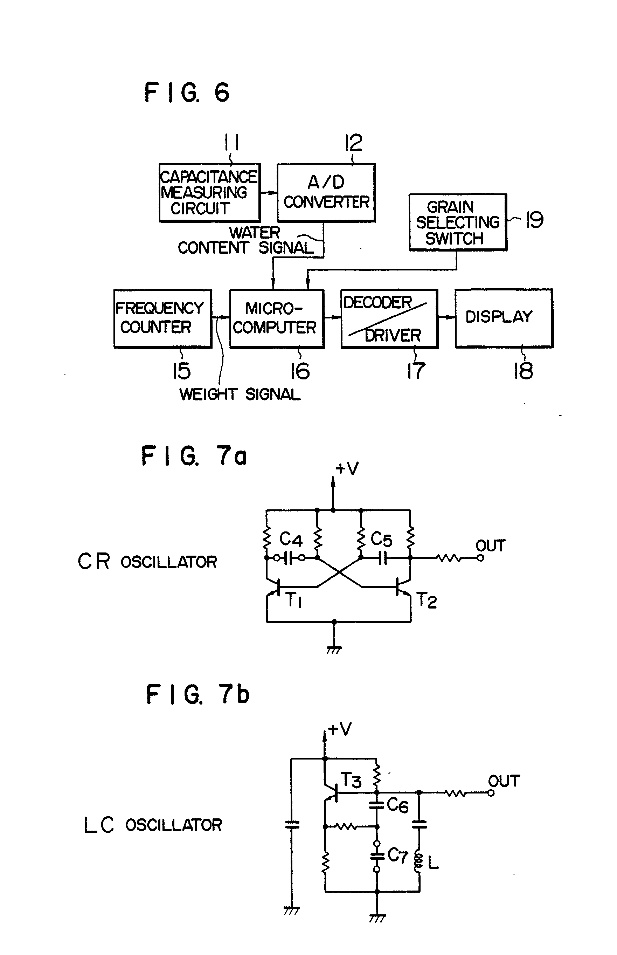

The load-to-capacitance converter 13 is constructed as shown in Fig. 5. That is, this converter 13 is formed of a spring balance comprising a fixed portion 53, springs 54 and 55 suspended from the fixed portion 53, and an upper electrode 51, the springs 54 and 55 being loaded with the gravitational force of the upper electrode 51 and the sample container 2, 3, 1, 9 shown in Fig. 3. The reciprocal distance between the upper electrode 51 and a lower electrode 52 is thus changed in proportion to the weight of the grain in the sample container. In other words, the weight of the grain is converted to the electric capacitance between the electrodes 51 and 52. The oscillator 14 can be formed of a known LC oscillator or RC oscillator. Figs. 7a and 7b are detailed circuit diagrams of the oscillator 14.

In Fig. 7a, a capacitance $C_4$ is the electric capacitance to which the weight of the grain is to be converted. This oscillator is formed of the capacitance $C_4$, a capacitor $C_5$, four resistors and two PNP transistors $T_1$ and $T_2$ and oscillates at a frequency proportional to the weight. In this case, NPN transistors may be used instead of the PNP transistors, to form a similar circuit.

A capacitance $C_7$ in Fig. 7b is the capacitance to which the weight is to be converted. This oscillation circuit is formed of the capacitance $C_7$, a coil L, a capacitance $C_6$ and an NPN transistor $T_3$ and oscillates at a frequency proportional to the weight. The oscillator 14 is connected to a frequency counter 15. Thus, the weight signal of a digital form produced from the frequency counter 15 is supplied to the microcomputer 16, where the measured electrical capacitance, $C_j$ (j = 1, 2, ...) shown in Fig. 1 is corrected for the deviation of the capacitance from the standard weight $W_s$.

The microcomputer 16 calculates the rate of water content from an equation derived from the following procedure:

(a) The standard weight $W_s$ for the sample weight of each sample to be measured, is determined;

(b) Samples of high, medium and low water content are prepared and the electric capacitances $C_1$, $C_2$, $C_3$, ... as shown in Fig. 2 are measured;

(c) The rates of water content M1, M2, M3, ... of the same samples are measured according to the standard method;

(d) A regression equation of the relation between the capacitance C the rate of water content M is derived from the above measured data as

$$M = f (C) \qquad (1)$$

where suitable f (C) is either a quadratic, or cubic form of C. Equations of fourth degree and above being normally not necessary; and

(e)

$$M = f \left( \frac{W_s}{W} C \right) \qquad (2)$$

from which the water content M corrected for the measured weight C can be determined.

Since the electric characteristics of different kinds of grain are different even if they have the same water content, a switch for selecting a kind of grain is added to the moisture meter, thereby making it more useful.

The moisture meter of this invention has a grain selecting switch 19 provided as shown in Fig. 6 (which shows part of Fig. 3). The microcomputer 16 calculates the water content M from the electric capacitance by using a coefficient corresponding to the electric signal indicative of a selected kind of grain supplied from the switch 19.

The same wheat sample as used in the measurement shown in Fig. 2 was measured by the moisture meter shown in Fig. 3 and the measured water content was corrected for the error due to the change of weight from the standard weight by using equation (2). The results are shown in Fig. 4. From Fig. 4, it will be seen that the measured data coincide with a curve 41 obtained in the same way as for the curve 31.

The rate of water content calculated by the microcomputer 16 is converted by a decoder/driver 17 to a data format suitable for being displayed, which is then displayed on a display 18.

**Claims**

1. An electric moisture meter for measuring the water content of grain, comprising:

   (a) a container (1, 2, 3, 9) with first and second electrodes (2, 3) between which a sample (4) of grain is placed;

   (b) a spring balance (51, 53, 54, 55) on which said container is placed and of which the springs (54, 55) expand down and contract up in proportion to the weight of said grain sample (4) and said container;

   (c) a fourth electrode (52) disposed to oppose a third electrode 51 mounted to said spring balance and thereby to form an electric capacitance between said third and fourth electrodes (51, 52), said fourth electrode being mounted to a fixed portion; and

   (d) means (12, 14, 15, 16) for correcting said water content corresponding to an electric capacitance measured between said first and second electrodes (2, 3) for error due to the change of weight from a standard weight by a voltage signal corresponding to the weight of said grain sample (4) which is measured between said third and fourth electrodes (51, 52).

2. An electric moisture meter according to Claim 1, wherein said correcting means comprises:

   an LC oscillator (14) which oscillates at frequency, corresponding to the weight of said sample, or determined by the electric capacitance ($C_7$) measured between said third and fourth (51, 52) electrodes and a fixed inductance;

   a frequency counter (15) for counting said frequency signal;

   means (12) for converting said electric capacitance measured between said first and second electrodes (2, 3) to a digital signal; and

   a correction arithmetic unit (16) which is responsive to the count from said frequency counter and said digital signal to calculate a corrected water content of said sample.

3. An electric moisture meter according to Claim 2, wherein said oscillator (14) has an inductance element (L) for receiving said electric capacitance signal.

4. An electric moisture meter according to Claim 2, wherein said oscillator (14) has resistance elements for receiving said electric capacitance signal.

**Revendications**

1. Hygromètre électrique pour la mesure de la teneur en eau de grains comportant :

   (a) un réceptacle (1, 2, 3, 9) comprenant une première et une deuxième électrodes (2, 3) entre lesquelles on place un échantillon (4) de grains ;

   (b) une balance à ressorts (51, 53, 54, 55) sur laquelle on place ledit réceptacle et dont les ressorts (54, 55) se détendent vers le bas et sont comprimés vers le haut proportionnellement au poids dudit échantillon de grains (4) et dudit réceptacle ;

   (c) une quatrième électrode (52) placée en opposition à une troisième électrode (51) montée sur ladite balance à ressorts pour constituer de la sorte une capacité électrique entre lesdites troisième et quatrième électrodes (51, 52) ladite quatrième électrode étant montée sur une partie fixe ; et

   (d) des moyens (12, 14, 15, 16) pour corriger ladite teneur en eau correspondant à une capacité électrique mesurée entre lesdites première et deuxième électrodes (2, 3) des erreurs dues à la variation de poids par rapport à un poids standard, par un signal de tension correspondant au poids dudit échantillon de grains (4) qui est mesuré entre lesdites troisième et quatrième électrodes (51, 52).

2. Hygromètre électrique selon la revendication 1, dans lequel ledit moyen de correction comporte :

un oscillateur (14) LC qui oscille à une fréquence correspondant au poids dudit échantillon, ou qui est déterminée par la capacité électrique ($C_7$) mesurée entre lesdites troisième et quatrième (51, 53) électrodes et une inductance fixe ;

un fréquencemètre (15) pour le comptage dudit signal de fréquence ;

un moyen (12) pour convertir ladite capacité électrique mesurée entre lesdites première et deuxième électrodes (2, 3) en un signal numérique ; et

une unité arithmétique de correction (16) qui est sensible au comptage effectué par ledit fréquencemètre et audit signal numérique, pour calculer une teneur en eau corrigée dudit échantillon.

3. Hygromètre électrique selon la revendication 2, dans lequel ledit oscillateur (14) comporte un élément d'inductance (L) pour recevoir ledit signal de capacité électrique.

4. Hygromètre électrique selon la revendication 2, dans lequel ledit oscillateur (14) comporte des éléments de résistance pour recevoir ledit signal de capacité électrique.

**Patentansprüche**

1. Elektrischer Feuchtigkeitsmesser zum Messen des Wassergehaltes von Korn, enthaltend:
a) einen Behälter (1, 2, 3, 9) mit ersten und Zweiten Elektroden (2, 3), zwischen den eine Kornprobe (4) angeordnet wird;
b) eine Federwaage (51, 51, 54, 55), auf die der Behälter aufgesetzt ist und deren Federn (54, 55) sich proportional zum Gewicht der Kornprobe (4) und des Behälters nach unten ausdehnen und nach oben zusammenziehen;
c) eine vierte Elektrode (52), die entgegengesetzt zur dritten Elektrode (51) angeordnet an der Federwaage befestigt ist und dadurch eine elektrische Kapazität zwischen den dritten und vierten Elektroden (51, 52) bildet und die an einen festen Abschnitt angebracht ist; und
d) eine Einrichtung (12, 14, 15, 16) zum Korrigieren des Wassergehaltes entsprechend einem zwischen den ersten und zweiten Elektroden (2, 3) gemessenen elektrischen Kapazität auf Fehler wegen der Änderung des Gewichts gegenüber einem Standardgewicht durch ein Spannungssignal, das dem Gewicht der Kornprobe (4) entspricht und das zwischen den dritten und vierten Elektroden (51, 52) gemessen wird.

2. Elektrischer Feuchtigkeitsmesser nach Anspruch 1, bei dem die Korrektureinrichtung enthält:

einen LC-Oszillator (14), der mit einer Frequenz schwingt, die dem Gewicht der Probe entspricht oder durch die elektrische Kapazität ($C_7$) zwischen den dritten und vierten Elektroden (51, 52) und einer festen Induktivität bestimmt wird;

einen Frequenzzähler (15) zum Zählen des Frequenzsignals;

eine Einrichtung (12) zum Umwandeln der zwischen den ersten und zweiten Elektroden (2, 3) gemessenen elektrischen Kapazität in ein digitales Signal; und

eine arithmetische Korrektureinheit (16), die auf das Zählergebnis des Frequenzzählers und das digitale Signal anspricht, um einen korrigierten Wassergehalt der Probe zu berechnen.

3. Elektrischer Feuchtigkeitsmesser nach Anspruch 2, bei dem der Oszillator (14) ein Induktanzelement (L) zur Aufnahme des elektrischen Kapazitätssignals aufweist.

4. Elektrischer Feuchtigkeitsmesser nach Anspruch 2, bei dem der Oszillator (14) Widerstandselemente zum Aufnehmen des elektrischen Kapazitätssignals aufweist.

F I G. 1

F I G. 2

F I G. 4

# F I G. 3

SAMPLE

PLASTIC PLATE 1

4

2 INNER ELECTRODE

3 OUTER ELECTRODE

INSULATING CASE 9

5

6

11 CAPACITANCE MEASURING CIRCUIT

12 A/D CONVERTER

LOAD-TO-CAPACITANCE CONVERTER 13

WATER CONTENT SIGNAL

7 8

OSCILLATOR 14

FREQUENCY COUNTER 15

MICRO-COMPUTER 16

DECODER / DRIVER 17

DISPLAY 18

WEIGHT SIGNAL

# F I G. 5

13

53 FIXED PORTION

54

51

29 INSULATING CASE

55

52

## F I G. 6

## F I G. 7a

CR OSCILLATOR

## F I G. 7b

LC OSCILLATOR